# EUROPEAN PATENT APPLICATION

(11) **EP 2 251 350 A2**
(43) Date of publication of application: **17.11.2010**
(21) Application number: 10167086.7
(22) Date of filing: 27.03.2006
(51) Int. Cl.: C07K 14/47, A61K 31/7088, A61K 31/7105, A61K 39/395, A61K 45/00, A61K 48/00, A61P 25/00, A61P 25/20, A61P 35/00, C12M 1/00, C12N 15/09

(54) **RORalpha promoting the induction of Bmal1**

(30) Priority: 29.03.2005 JP 2005096459
(62) Divisional of application: 06730113.5
(71) Applicant: Sony Corporation, Minato-ku Tokyo 108-0075 (JP)
(72) Inventor: Yamamoto, Takuro, Minato-Ku Tokyo (JP); Mamine, Takayoshi, Minato-Ku Tokyo (JP); Takumi, Toru, Minato-Ku Tokyo (JP); Akashi, Makoto, Minato-Ku Tokyo (JP)
(74) Representative: Keston, Susan Elizabeth

(57) **Abstract**

An object of the present invention is to clarify unelucidated aspects in the control mechanism of circadian rhythms. The present inventors have newly found that RORα (retinoic acid binding-receptor alpha; the same shall apply hereinafter) stimulates an induction of Bmall expression and also that the induction of Bmall expression is promoted under hypoxia. These findings strongly suggest the existence of a control mechanism of circadian rhythms that, when RORα expression is promoted under hypoxia or the like, an induction of Bmal1 expression is stimulated and, when the induction of Bmal1 expression is stimulated, binding between BMAL1 and CLOCK is stimulated and an induction of Per gene or Cry gene expression is stimulated. The present invention, therefore, has applicability as jet-lag regulating agents and anticancer agents.

## Description

### [Technical Field]

This invention relates to an acting mechanism of a clock gene and its peripheral technologies. More specifically, the present invention is concerned with RORα (retinoic acid receptor-related orphan receptor) capable of stimulating an induction of Bmal 1 (brain-muscle ARNT-like protein 1) expression, anticancer agents, jet-lag regulating agents, screening methods for these drugs, etc.

### [Background Art]

Circadian rhythms are biorhythms having a cycle of about 24 hours, and are in vivo phenomena that can be commonly observed in numerous organisms ranging from unicellular organisms to human beings. Each organism has genes which take part in the control of circadian rhythms, and these circadian rhythm control genes perform regulation of the circadian rhythms.

In mammals, circadian rhythm control genes are particularly high in expression abundance at suprachiasmatic nucleus (SCN) of the hypothalamus, and play a central role in the regulation of circadian rhythms on the individual level. In other in vivo tissues (circadian rhythm peripheral tissues), on the other hand, circadian rhythm control genes are also expressed, and like the center of circadian rhythms, the regulation of circadian rhythms is performed by the circadian rhythm control genes in every cell and every tissue.

Known as circadian rhythm control genes in mammals are Per genes (per1, Per2, Per3), Clock gene, Bmal genes (Bmal1, Bmal2, Bmal3), Cry gene, Rev-erb genes (Rev-erbα, Rev-erbβ), and the like.

Among these, BMAL1 (a transcript of Bmal1 gene; the same shall apply hereinafter) has been suggested to form a dimer with CLOCK (a transcript of Clock gene; the same shall apply hereinafter) and to activate the expression of Per gene, Cry gene or the like via E-box (the promoter region of Per1 gene) (see Gekakis, N. et al. "Role of the CLOCK protein in the mammalian circadian mechanism" Science 280, 1564-9 (1998), etc.).

It has also been suggested that the expression of Bmal1 gene is regulated by REV-ERB (a transcript of Rev-erb gene; the same shall apply hereinafter) or the like (see Preitner, N. et al. "The orphan nuclear receptor REV-ERBalpha controls circadian transcription within the positive limb of the mammalian circadian clock" Cell 110, 251-60 (2002), etc.).

A description will now be made about RORα (retinoic acid binding-receptor alpha; the same shall apply hereinafter), which is a protein according to the present invention. RORα is a protein also called "RORA (Rar-related orphan receptor A)" or the like, and has been found to include splicing variants such as RORα1 and RORα4. It is to be noted that the term "splicing variant" means a protein derived from the same gene but different in a part of its amino acid sequence and that it occurs due to a difference in the manner of joining upon splicing.

It has also been reported that the expression of RORα promoted under hypoxia (see Chauvet, C. et al. "Retionic acid receptor-related orphan receptor (ROR)alpha4 is the predominant isoform of the nuclear receptor RORalpha in the liver and is up-regulated by hypoxia in HepG2 human hepatoma cells" Biochem J364, 449-56 (2002)).

Although the control mechanism of circadian rhythms especially in mammals has not been elucidated yet in many aspects, circadian rhythms have been suggested to be deeply linked not only with sleep and vigilance but also with the homeostasis and in vivo regulatory mechanism of organisms, such as thermoregulation and hormonal secretions. In addition, circadian rhythms have a potential link with the onset mechanisms of many diseases led by cancers.

It is, therefore, a principal object of the present invention to clarify such unelucidated aspects in the control mechanism of circadian rhythms.

### [Disclosure of Invention]

The present inventors have newly found that RORα (retinoic acid binding-receptor alpha; the same shall apply hereinafter) stimulates an induction of Bmal1 expression and also that the induction of Bmal1 expression is promoted under hypoxia.

The present invention, therefore, provides RORα capable of stimulating an induction of Bmal1 expression, and RORα capable of promoting the induction of Bmal1 expression under hypoxia.

The present invention strongly suggests the existence of a circadian rhythm regulating mechanism such as that to be described next.

When the expression of RORα is promoted under hypoxia or the like, the induction of Bmall expression is stimulated. When the induction of Bmall expression is stimulated, the binding between BMAL1 and CLOCK is stimulated and an induction of Per gene and/or Cry gene expression is stimulated.

It has also been newly found in the present invention that RORα stimulates the induction of Bmal1 expression competitively with REV-ERBα or REV-ERBβ. Accordingly, the present invention also strongly suggests the existence of a mechanism that regulates circadian rhythms as a result of control of the induction of Bmal1 expression by both RORα and REV-ERB.

By the present invention, interactions between RORα and the promoter regions (ROREs) of Bmal1 have been elucidated. Therefore, circadian rhythms can be regulated by the present invention when a modulation of the circadian rhythms is induced by a modulation or the like of the rhythm of Bmal1 expression.

For example, a substance that represses RORα has applicability as a jet-lag regulating agent. Other cases associated with modulations of circadian rhythms include, for example, sleep disorders, vigilance disorders, insomnia, vegetative syndromes, depression, senile dementia, deteriorations of shape through disruptions of living rhythms such as nightshift and shiftwork, exhaustions through disruptions of living rhythms in autism and the like, and so on. The present invention is also equipped applicability to these diseases.

Moreover, the present invention is also equipped with applicability as an anticancer agent.

In general, cancer tissues often fall in hypoxia. Further, concerning an increase in the onset rate of breast cancers, for example, among nurses through nightshift, modulations in circadian rhythms have been indicated to be its incitements.

In this respect, It has been ascertained in the present invention that the induction of Bmal1 expression is promoted under hypoxia. A substance that represses RORα is, therefore, equipped with applicability as an anticancer agent.

Moreover, the present invention is also applicable to DNA chips, protein chips, use as markers for the detection of modulations of circadian rhythms, screening methods for anticancer agents or jet-lag regulating agents, etc.

By the present invention, it has become evident that RORα stimulates the induction of Bmal1 expression. Circadian rhythms can, therefore, be regulated based on this finding.

### [Brief Description of Drawings]

[FIG. 1] FIG. 1 is a diagram showing a control mechanism of a circadian rhythm.
[FIG. 2] FIG. 2 diagrammatically illustrates expression patterns of Reverbα, Rorα and Bmal1 in various tissues.
[FIG. 3] FIG. 3 diagrammatically illustrates expression patterns of Reverbα, Rorα and Bmal1 in culture cells NIH3T3.
[FIG. 4] FIG. 4 is a diagram depicting that RORα induced Bmal expression.
[FIG. 5] FIG. 5 is a diagram showing that the induction of Bmal expression is also stimulated as the expression abundance of RORα increases.
[FIG. 6] FIG. 6 is a diagram illustrating that RORα and REV-ERB protein compressively act on the promoter regions of Bmal.
[FIG. 7] FIG. 7 diagrammatically shows circadian variations of the expression abundance of Bmal when cells were cultured in the presence of a dominant-negative mutant of RORα.
[FIG. 8] FIG. 8 depicts the sequences of prepared recombinant vectors.
[FIG. 9] FIG. 9 is a diagram showing induction levels of Bma1 expression by RORα when the length of an upstream region of Bmal1 was changed.
[FIG. 10] FIG. 10 diagrammatically illustrates circadian variations of the expression abundance of Bmal1 when the length of the upstream region of Bmal1 was changed.
[FIG. 11] FIG. 11 shows a photograph of a western blot panel by a pull-down assay (photograph substituted for drawing).
[FIG. 12] FIG. 12 shows actograms when subjected to free running under dark conditions after light conditions and the dark conditions were repeated alternately for 12 hours over 15 days.
[FIG. 13] FIG. 13 is a diagram depicting circadian cycles of mice as acquired when the rearing environment was set to dark conditions for 24 hours after light conditions and the dark conditions were repeated alternately for 12 hours over 15 days.
[FIG. 14] FIG. 14 shows actograms when the starting time of dark conditions was advanced by 4 hours to create a state having a time difference after light conditions and the dark conditions were repeated alternately for 12 hours over 15 days.
[FIG. 15] FIG. 15 depicts actograms when environmental conditions were changed to an environment consisting of light conditions and dark conditions repeated alternately for 6 hours after the light conditions and the dark conditions were repeated alternately for 12 hours over 15 days.
[FIG. 16] FIG. 16 diagrammatically illustrates the induction of Bmal expression when the expression of RORα was repressed by RNA interference.
[FIG. 17] FIG. 17 is a diagram showing circadian variations of the induction of Bmal expression when an RORα-gene knockout mutant of mouse embryonic fibroblast was used.
[FIG. 18] FIG. 18 is a diagram showing circadian variations of the expression abundance of Bmal as determined by quantitative realtime RT-PCR when an RORα-gene knockout mutant of mouse embryonic fibroblast was used.
[FIG. 19] FIG. 19 diagrammatically illustrates the induction of Bmal expression when the expression of RORα was repressed using an antisense.
[FIG. 20] FIG. 20 is a diagram depicting that the induction of Bmal1 expression was promoted under hypoxia.

### [Best modes for Carrying out the Invention]

### <RORα according to the present invention>

Firstly, a description will hereinafter be made about RORα according to the present invention.

RORα according to the present invention has splicing variants such as RORα1 and RORα4. The amino acid sequences of RORα1 and RORα4 in human being are described under SEQ ID NO: 1 and SEQ ID NO: 2, respectively. The amino acid sequences of RORα1 and RORα4 in mouse, on the other hand, are described under SEQ ID NO: 3 and SEQ ID NO: 4, respectively.

It is to be noted that RORα according to the present invention has homology to RORα1, RORα4, or the amino acid sequence of any one of the above-described proteins, and is a protein having a region that interacts with the promoter regions (ROREs) of Bmal1. For example, other splicing variants of one or more of the above-described proteins and those containing a substituted, deleted, inserted or added region in parts of the amino acid sequences of one or more of the above-described proteins are hence included in the term "RORα" according to the present invention.

### <Circadian rhythm regulating mechanism according to the present invention>

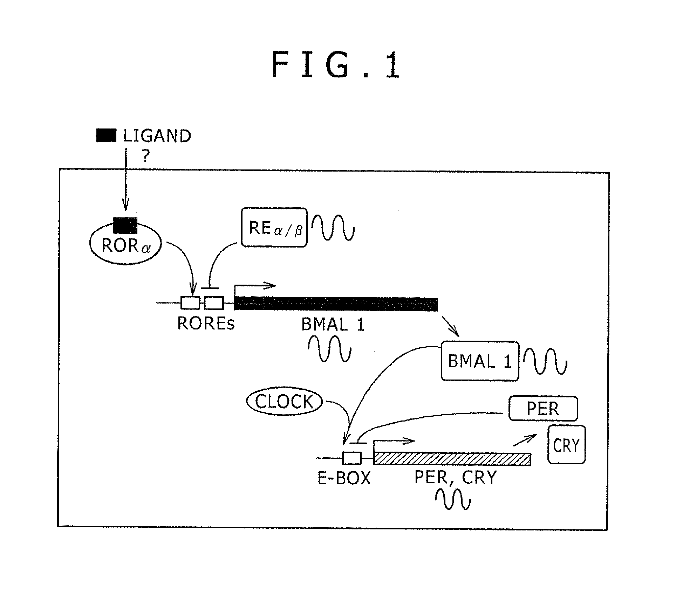

Referring next to FIG. 1, a description will hereinafter be made about the RORα-dependent regulating mechanism for a circadian rhythm.

It has been ascertained by the present invention that RORα binds to the promoter regions (ROREs) of Bmal1 and stimulates the induction of Bmal1 expression. A circadian rhythm regulating mechanism such as that to be described next is, therefore, presumed to exist.

When the expression of RORα is promoted under hypoxia or the like, RORα acts on the promoter regions (ROREs) of Bmal1 to stimulate the induction of Bmal1 expression. BMAL1 so expressed forms a dimer with CLOCK to stimulate the induction of Per gene or Cry gene expression via E-box (the promoter region of Per gene, Cry gene or the like). Thus thus-expressed PER (a transcript of Per gene) or CRY (a transcript of Cry gene) directly takes part in the formation of the circadian rhythm.

It is to be noted that in the figure, "REα/β" indicates REV-ERB. REV-ERB is considered to bind to the promoter regions (ROREs) of Bmal1 competitively with RORα and to repress the expression of Bmal1.

On the other hand, "Ligand" in the figure is a substance that stimulates or represses the expression of RORα. As described above, the expression of RORα is deeply linked to a regulation of a circadian rhythm. By conducting a screening for the substance (ligand), it is possible to make a search for a novel jet-lag regulating agent, anticancer agent or the like. A screening system can, therefore, be designed based on the circadian rhythm regulating mechanism according to the present invention.

### <Substances capable of repressing RORα according to the present invention>

A description will hereinafter be made about substances capable of repressing RORα according to the present invention (anticancer agents, jet-lag regulating agents and the like, each of which comprises at least an RORα-repressing substance).

In Examples to be described subsequently herein, results were obtained to the effect that, when a circadian rhythm is shifted (for example, when the starting time of light conditions is changed), a repression of RORα makes it possible to synchronize the circadian rhythm to a new circadian rhythm earlier than conventional approaches. Substances which can repress RORα according to the present invention are, therefore, applicable as jet-lag regulating agents.

It has also been ascertained in the present invention that the induction of Bmal1 expression can be promoted under hypoxia. On the other hand, cancer tissues are known to fall under hypoxia as mentioned above. The substances which can repress RORα according to the present invention are, therefore, applicable as anticancer agents.

Examples of such RORα-repressing substances include anti-RORα antibodies, and siRNAs, antisense nucleic acids and the like containing parts of the coding sequence of RORα.

The anti-RORα antibodies bind specifically to RORα to repress the function of RORα.

siRNA (short interfering RNA) is a double-stranded RNA, which represses gene expression by RNA interference. siRNAs which contain parts of the coding sequence of RORα repress the expression of RORα, and therefore, can repress the function of RORα.

The antisense nucleic acids are nucleic acids which contain parts of the coding sequence of RORα. The term "antisense nucleic acid" broadly embraces those synthesized artificially and those subjected to modification, treatment and/or the like for stabilization. The antisense nucleic acids suppress the expression of RORα by their complementary binding to a transcript (mRNA) of RORα, and therefore, can repress the function of RORα.

The anti-RORα antibodies, siRNAs and antisense nucleic acids can each be prepared by a known method.

### <DNA chip and protein chip according to the present invention>

The DNA chip and protein chip according to the present invention will hereinafter be described.

A nucleic acid having the base sequence of the gene, which encodes RORα, or a nucleic acid having a part of the base sequence of the gene can be applied to a DNA chip. Immobilization of such a nucleic acid on the DNA chip makes it possible to detect the expression abundance of RORα, for example, in blood or a given tissue, so that the resulting DNA chip can be useful for the detection of a modulation or the like of a circadian rhythm. The immobilization of the nucleic acid on the DNA chip can be conducted by a method known *per se* in the art.

Similarly, an antibody which specifically binds to RORα is applicable to a protein chip. Immobilization of this antibody on the protein chip makes it possible to detect the expression abundance of RORα, for example, in blood or a given tissue, so that the resulting protein chip can be useful for the detection of a modulation or the like of a circadian rhythm. The immobilization of the antibody on the protein chip can also be conducted by a method known per se in the art.

### <Use according to the present invention as a marker for the detection of a modulation of a circadian rhythm>

A description will hereinafter be made about the use according to the present invention as a marker for the detection of a modulation of a circadian rhythm.

RORα according to the present invention can be useful as a marker for the detection of a modulation of a circadian rhythm, because it plays an important role in a mechanism that regulates the circadian rhythm. Specifically, a modulation of a circadian rhythm can be detected by detecting the expression abundance of RORα, for example, in blood or a given tissue in accordance with ELISA (known method) and determining a modulation in the expression abundance of RORα.

### <Screening method according to the present invention>

A description will hereinafter be made about an example of the screening method according to the present invention.

When conducting screening to search for a substance that represses RORα, a target substance can be searched out, for example, by detecting an interaction between RORα and the promoter regions (ROREs) of Bmal1 after feeding the target substance to a reaction system. Specifically, when the interaction is repressed by feeding the target substance to the reaction system, the target substance can be useful as an anticancer agent or jet-lag regulating agent.

Specific examples of the screening method can include a method which includes immobilizing beforehand an oligonucleotide of the promoter regions (ROREs) of Bmal1 on a column, beads or the like, feeding RORα and a target substance, and then detecting an interaction between RORα and the promoter regions (ROREs) of Bmal1. In this case, it is possible to apply, for example, a coimmunoprecipitation assay, a pull-down assay making use of a column or beads, or the like as a method for the detection of the interaction.

It is to be noted that the screening method according to the present invention can be implemented into a kit. In this case, for example, RORα, the oligonucleotide of the promoter regions (ROREs) of Bmal1, an anti-RORα antibody, an interaction detecting reagent (a reagent useful in a coimmunoprecipitation assay, a pull-down assay making use of a column or beads, or western blotting) and the like may be combined into a kit as needed. As the protein and oligonucleotide, their mutants may be used in the above case.

### [Example 1]

In Example 1 and Example 2, expression patterns of Rev-erbα, Rorα and Bmal1 were investigated as preliminary experiments.

In Example 1, plural tissues were collected from mice, and the expression patterns of Rev-erbα, Rorα and Bmal1 in those tissues were investigated.

An outline of experimental procedure was as follows.
(1) Firstly, the circadian rhythms of the mice employed in the experiments were synchronized. The mice were reared for 2 weeks in a room controlled under light conditions from 8:00 until 20:00 and under dark conditions from 20:00 until 8:00 on the following day so that the circadian rhythms of the mice were synchronized.
(2) As circadian rhythm peripheral tissues, the liver, heart, kidney, lung and stomach were next collected at predetermined times from the mice. It is to be noted that the collection times of each organ were set at 4-hour intervals from immediately after the synchronization of the circadian rhythms (8:00, 12:00, 16:00, 20:00, 24:00, 4:00 on the following day).
(3) Subsequent to homogenization of each organ so collected, total RNA was extracted from the organ by using "Promega total SV RNA Isolation Kit" (product of Promega Corporation).
(4) Quantitative realtime RT-PCR (quantitative realtime reverse transcriptase polymerase chain reaction) was then conducted to measure the expression abundances of the respective genes Rev-erbα, Rorα and Bmal1.

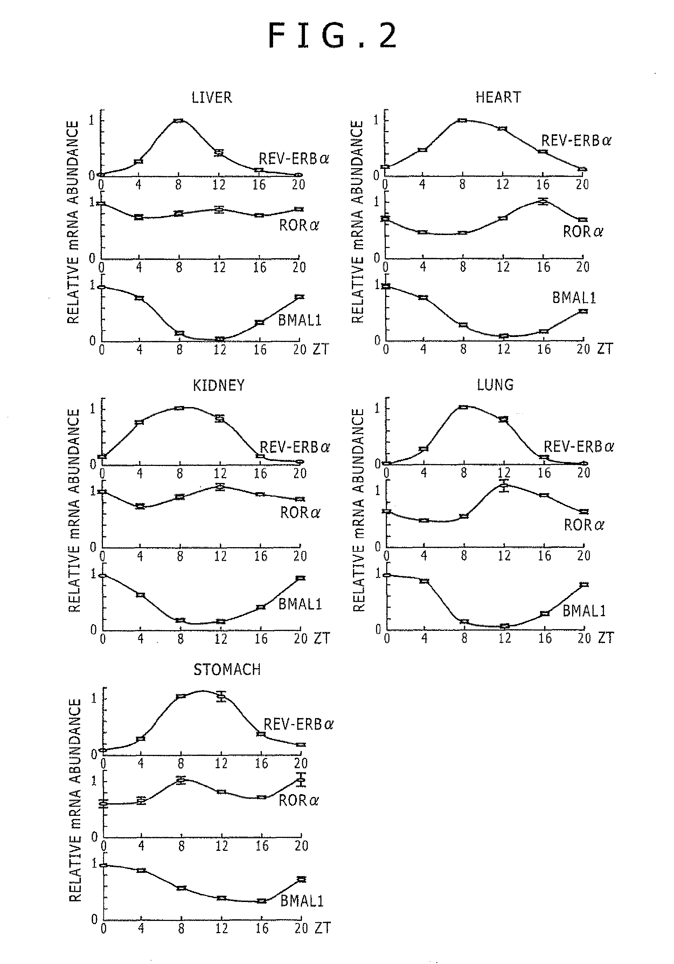

The results are shown in FIG. 2.

In the figure, "liver" indicates the liver, "heart" the heart, "kidney" the kidney, "lung" the lung, and "stomach" the stomach, respectively.

In each diagram, the abscissa "ZT(Zeitgeber Times)" represents the circadian time, while the ordinate ("relative mRNA abundance" represents the relative expression abundance (expression level). It is to be noted that values of the expression abundance along the ordinate are relative values when the expression abundance of a housekeeping gene GAPDH (GlycerAldehyde-3-Phosphate DeHydrogenase) was assumed to be 1.

### [Example 2]

In Example 2, expression patterns of Rev-erbα, Rorα and Bmal1 in culture cells NIH3T3 were investigated by a similar procedure as in Example 1.

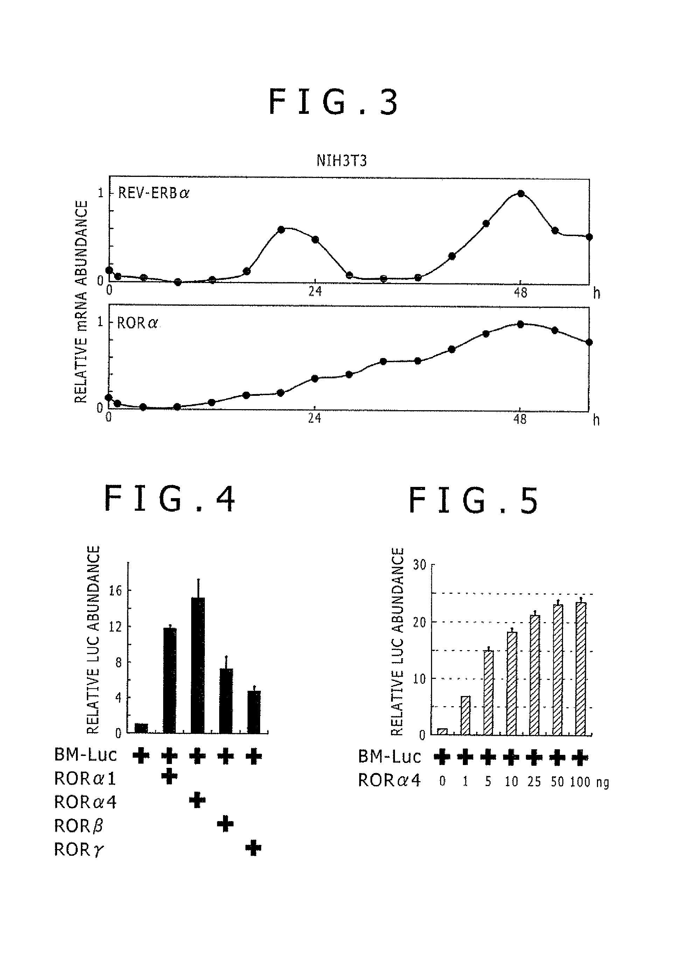

The results are shown in FIG. 3.

In the figure, the abscissa represents the circadian time, while the ordinate ("relative mRNA abundance" represents the relative expression abundance (expression level). It is to be noted that as in Example 1, values of the expression abundance along the ordinate are relative values when the expression abundance of the housekeeping gene GAPDH (GlycerAldehyde-3-Phosphate DeHydrogenase) was assumed to be 1.

From Example 1 and Example 2, the expression patterns of the three genes Rev-erbα, Rorα and Bmal1 in the respective tissues have been successfully ascertained.

### [Example 3]

Example 3 and Example 4 are directed to experiments in which investigations were conducted by a luciferase assay as to whether or not RORα would induce the expression of Bmal1.

A description will hereinafter be made about an outline of the "luciferase assay".

Luciferase is an enzyme protein which catalyzes bioluminescence.

Assume, for example, that the promoter region of a predetermined gene and a gene encoding luciferase are fused to prepare a recombinant gene and the recombinant gene is inserted in cultured cells. If an expression of the gene is induced, luciferase is expressed.

It is, therefore, possible to estimate the induction level of the expression of the gene by measuring luciferase-dependent bioluminescence and acquiring an expression level of luciferase.

In Example 3, an investigation was conducted by the luciferase assay as to whether or not ROR family proteins (RORα1, RORα4, RORβ, RORγ) would induce the expression of Bmal1.

An outline of experimental procedure will hereinafter be described.
(1) Firstly, the promoter regions of Bmal1 were inserted into a luciferase luminescence vector "pGL3 basic" (product of Promega Corporation) to prepare a recombinant vector. That recombinant vector contained the promoter regions of Bmal1 and a region encoding the luciferase gene.
(2) Further, DNA sequences that encode the ROR family proteins, respectively, were separately inserted into an expression vector "pcDNA3" (product of Invitrogen Japan K.K.) to prepare recombinant vectors.
(3) Then, the recombinant vectors prepared in the steps (1) and (2) were cotransfected into culture cells NIH3T3. The thus-transfected culture cells were next isolated and subcultured to coexpress both of the proteins. Thirty-six hours after the transfection, the subcultured cells were collected and lyzed.
(4) Using "Dual Luciferase Assay System" (product of Promega Corporation), a solutions of the lyzed cells was next prepared. Thereafter, luminescence intensities were measured to acquire induction levels of Bmal1 expression, respectively.

The results are shown in FIG. 4.

In the figure, "BM-Luc" indicates the recombinant vector containing the promoter regions of Bmal1 and the region encoding the luciferase gene, "RORα1" the recombinant vector with the RDRα1-encoding DNA sequence inserted therein, "RORα4" the recombinant vector with the RORα4-encoding DNA sequence inserted therein, "RORβ" the recombinant vector with the RORβ-encoding DNA sequence inserted therein, and "RORγ" the recombinant vector with the RORγ-encoding DNA sequence inserted therein, respectively, and "+" represents the transfection of those recombinant vectors in the culture cells.

The ordinate "relative luc activity" represents the luminescence intensity. Its values are relative values when the luminescence intensity upon transfection of only "BM-Luc" in the culture cells was assumed to be "1".

The results shown in FIG. 4 strongly suggest that the ROR family proteins, especially RORα1 and RORα4 induce the expression of Bmal1.

### [Example 4]

In Example 4, an investigation was conducted as to whether or not the induction level of Bmal1 expression would also vary when the expression level of RORα4 is varied.

The experiment was conducted by a similar procedure as in Example 3.

It is to be noted that, when the recombinant vector with the RORα4-encoding DNA sequence inserted therein was transfected into culture cells, the amount of the recombinant vector was set at 0 (control), 1, 5, 10, 25, 50 and 100 ng, respectively.

The results are shown in FIG. 5.

In the figure, "BM-Luc" indicates the recombinant vector containing the promoter regions of Bmall and the region encoding the luciferase gene, and "+" represents the transfection of the recombinant vector in the culture cells.

"RORα4" indicates the recombinant vector with the RORα4-encoding DNA sequence inserted therein, and the numbers represent the amounts (unit: ng) of the recombinant vector added upon transfection.

The ordinate "relative luc activity" represents the luminescence intensity. Similar to FIG. 4, its values are relative values when the luminescence intensity upon transfection of only "BM-Luc" in the culture cells was assumed to be "1".

The results of FIG. 5 strongly suggest that the induction of Bmal1 expression is stimulated with an increase in the expression abundance of RORα4. This result, therefore, strongly suggests that a stimulation of RORα4 induces the expression of Bmal1.

### [Example 5]

The Rev-erb gene is known to be a circadian rhythm control gene, and the REV-ERB protein is known to bind to the promoter regions (ROREs) of Bmall to repress the expression of Bmal1.

In Example 5, an investigation was hence conducted as to whether or not the expression of Bmal1 would be induced when the RORα protein and the REV-ERB protein are coexpressed.

An outline of experimental procedure was as follows.
(1) Firstly, a recombinant vector with the promoter regions of Bmal1 inserted therein, a recombinant vector with the RORα1-encoding DNA sequence inserted therein and a recombinant vector with the Rev-erb gene inserted therein were prepared by similar procedure as in Example 3, etc., respectively.
(2) Then, those three recombinant vectors were co-transfected into culture cells NIH3T3 to co-express the respective proteins.
(3) After the cultured cells were lyzed, a solution of the lyzed cells was prepared using "Dual Luciferase Assay System" (product of Promega Corporation), and luminescence intensities were measured.

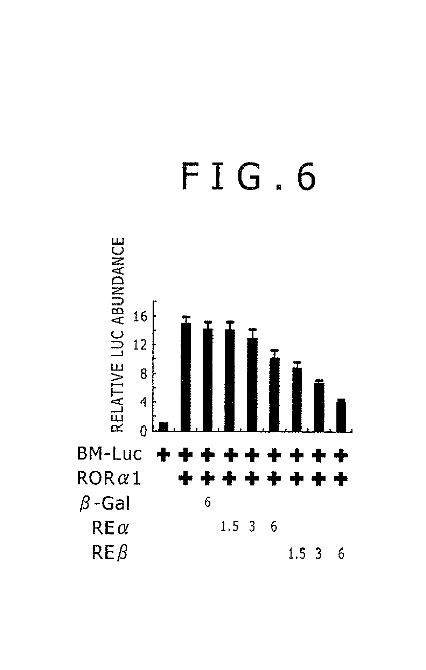

The results are shown in FIG. 6.

In the figure, "BM-Luc" indicates the recombinant vector containing the promoter regions of Bmal1 and the region encoding the luciferase gene, and "+" represents the transfection of those recombinant vectors in the culture cells.

"RORα1" indicates the recombinant vector with the RORα1-encoding DNA sequence inserted therein, and the parenthesized numbers indicate the amounts (unit: ng) of the vectors added upon transfection.

"β-Gal" indicates coexpression of β-galactosidase by similar procedure as that described above, and is a control. The number indicates the amount (unit: ng) of the recombinant vector added upon transfection.

"REα" and "REβ" indicate the recombinant vectors with the Rev-erbα gene and the Rev-erbβ gene inserted therein, respectively, and the numbers of the amounts (unit: ng) of the corresponding recombinant vectors added upon transfection.

The results of FIG. 6 strongly suggest that RORα and the REV-ERB protein competitively act on the promoter regions of Bmal1.

### [Example 6]

In Example 6, an investigation was conducted about circadian variations of Bmal1 expression abundance when cells were cultured in the presence of a dominant-negative mutant of RORα.

An outline of experimental procedure was as follows.

Similarly to Example 3, etc., the promoter regions of Bmall were firstly inserted into the luciferase luminescence vector "pGL3 basic" (product of Promega Corporation) to prepare a recombinant vector.

The recombinant vector was next transfected into culture cells NIH3T, and the resulting culture cells were then isolated and subcultured.

A serum shock was then applied to the culture cells to generate a circadian rhythm.

After the dominant-negative mutant of RORα1 was added to the resultant culture liquor, luciferin (a substrate for luciferase) was added at 15-minute intervals to measure luciferase activities (luminescence intensities). From circadian variations of the luminescence intensity, a circadian rhythm of Bmal1 transcription was acquired.

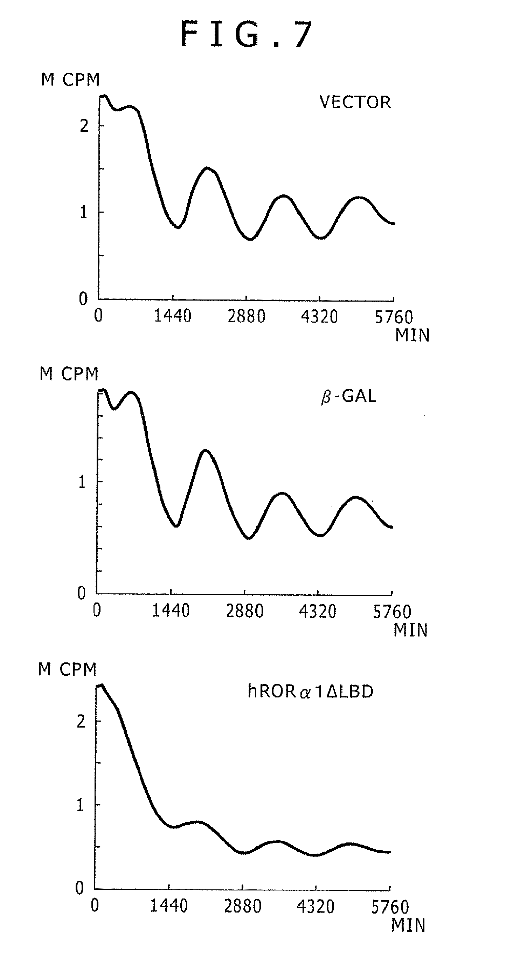

The results are shown in FIG. 7.

In the figure, the abscissa represents the time (unit: min), while the ordinate represents the luminescence intensity (unit: Mcpm, Million of Counts Per Minutes).

"vector" indicates the experimental results when an empty vector (pcDNA3) was added in place of the dominant-negative mutant (control).

"β-Gal" indicates the experimental results when β-galactosidase was added in place of the dominant-negative mutant (control).

"hRORα1ΔLBD" indicates the experimental results when the dominant-negative mutant of human RORα1 was added.

As illustrated in FIG. 7, the circadian rhythm of Bmal1 expression was repressed when the dominant-negative mutant of RORα1 was added. This result, therefore, strongly suggests that RORα not only induces the expression of bmal1 but also plays an important role in the generation of the circadian rhythm of Bmal1 transcription.

### [Example 7]

In Example 7 and Example 8, investigations were conducted for an RORα-acting site in the Bmal1 sequence upon induction of Bmal1 expression by RORα.

In Example 7, as upstream-region DNA sequences of the Bmal1 gene, those of different lengths were prepared, and by a similar procedure as in Example 3, etc., induction levels of Bmal1 expression by RORα were acquired.

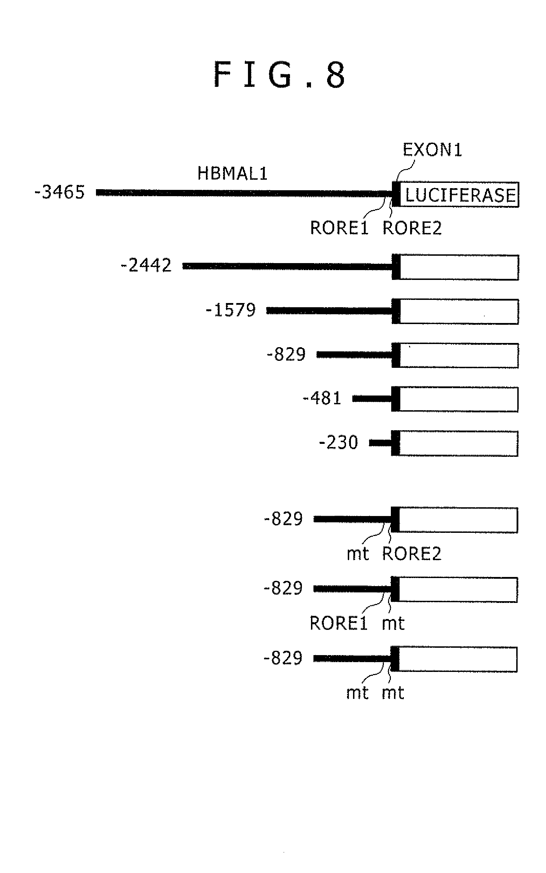

A description will hereinafter be made about an outline of experimental procedure.
(1) Firstly, recombinant vectors having the DNA sequences shown in FIG. 8 were prepared, respectively, by using the luciferase luminescence vector "pGL3 basic" (product of Promega Corporation).
   In the figure, "Luciferase" indicates that the region is a luciferase-encoding DNA sequence, and "Exon1" indicates that the region has the sequence of the first exon region in the Bmal1 gene. It is to be noted that "hBmal1" represents the human Bmal1 gene.
   "RORE1" and "RORE2" indicate that the regions are the promoter regions of the Bmal1 gene.
   "-3465" indicates that the DNA sequence has a sequence to an upstream by as many as 3465 bases in the Bmal1 gene sequence.
   "mt" represents a mutant, and indicates that RORE1, RORE2 or both of them mutated in part.
(2) After RORα1 and luciferase were coexpressed by similar procedure as in Example 3, etc., the luminescence intensity was measured to acquire an induction level of Bmal1 expression.

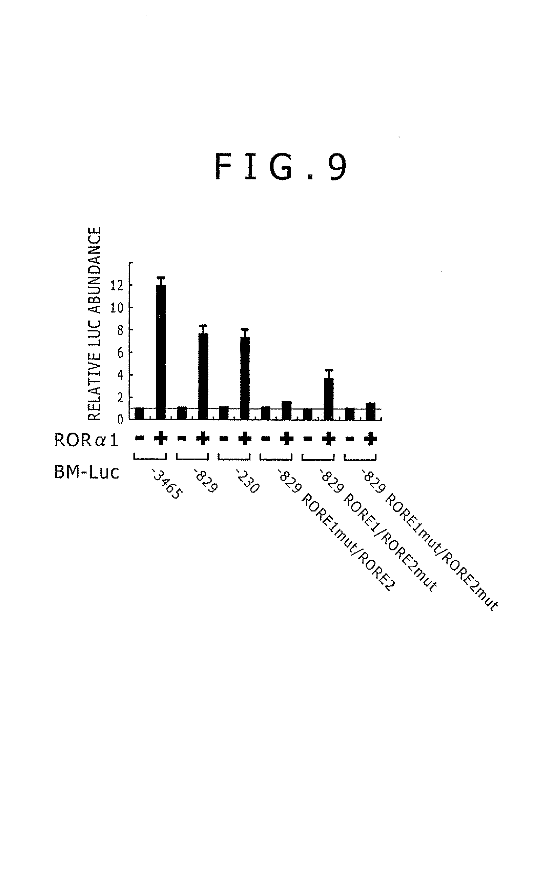

The results are shown in FIG. 9.

In the figure, "RORα1" indicates a recombinant vector with the RORα1-encoding DNA sequence inserted therein, "+" represents that the recombinant vector had been transfected into culture cells, and "-" represents that the recombinant vector had not been transfected into culture cells.

"BM-Luc" indicates the recombinant vector containing the promoter regions of Bmal1 and the region encoding the luciferase gene.

"-3465" indicates that the recombinant vector has a sequence to an upstream by as many as 3465 bases in the Bmal1 gene sequence, "-829" indicates that the recombinant vector has a sequence to an upstream by as many as 829 bases in the Bmall gene sequence, and "-230" indicates that the recombinant vector has a sequence to an upstream by as many as 230 bases in the Bmal gene sequence.

Further, "RORE1mut/RORE2" represents the deletion of RORE1, "RORE1/RORE2mut" the deletion of RORE2, and "RORE1mut/RORE2mut" the deletion of both RORE1 and RORE2. It is to be noted that "mut" represents a mutant.

### [Example 8]

In Example 8, an investigation was conducted in accordance with a similar procedure as in Example 6, etc. about circadian variations of Bmal1 expression abundance by using the recombinant vectors prepared in Example 7 (see FIG. 8).

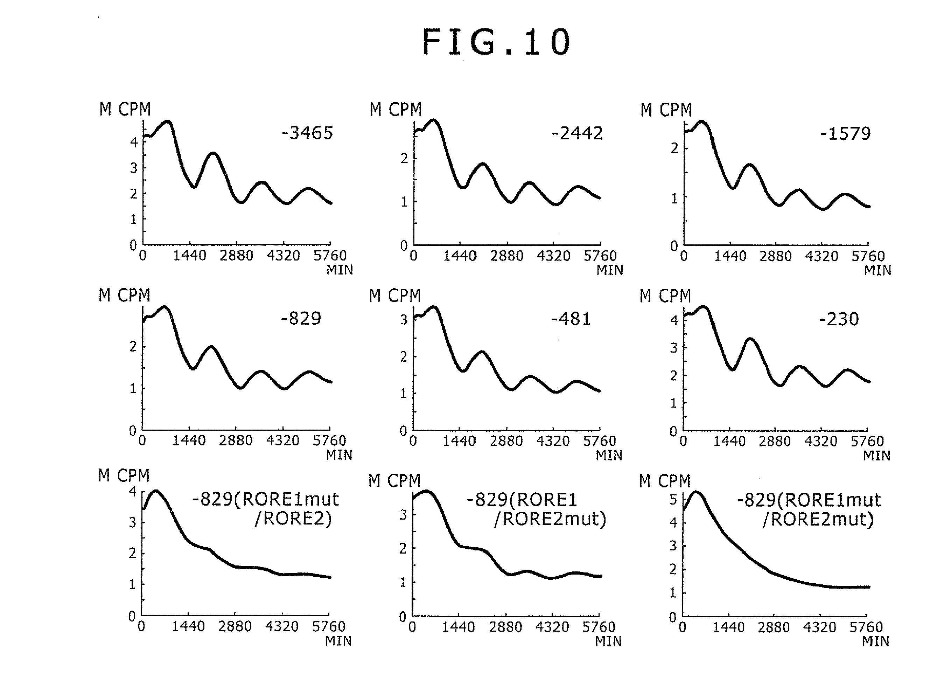

The results are shown in FIG. 10.

The labels in the figure have similar meanings as in FIG. 7.

The results of Example 7 (FIG. 9) and Example 8 (FIG. 10) indicate that the induction of Bmal1 expression by RORα requires both promoter regions (RORE1, RORE2) of the Bmal1 gene. It is, therefore, strongly suggested that RORα acts on both promoter regions of the Bmal1 gene to induce the expression of Bmal1.

### [Example 9]

In Example 9, binding between the promoter regions (ROREs) of Bmal1 and RORα was investigated by a pull-down assay.

An outline of experimental procedure is as follows.
(1) Firstly, subsequent to the collection of the liver from a mouse at a predetermined time, the liver was homogenized to obtain an extract of the mouse liver.
(2) Further, an oligonucleotide which contained the sequences of the promoter regions (ROREs) of Bmal1 was biotinylated. The biotinylated oligonucleotide was immobilized on "Streptoavidin-Sepharose Beads" (product of Amersham Biosciences K.K.).
(3) The beads (2) were then added to the mouse liver extract obtained in the step (1), followed by incubation.
(4) Subsequent to recovery (pull-down) of the beads, western blotting was performed with an anti-RORα antibody to detect RORα.

By the above procedure, it is possible to investigate whether or not an oligonucleotide, which contains the sequences of the promoter regions (ROREs) of Bmal1, and RORα, which is contained in a liver extract, are bound together.

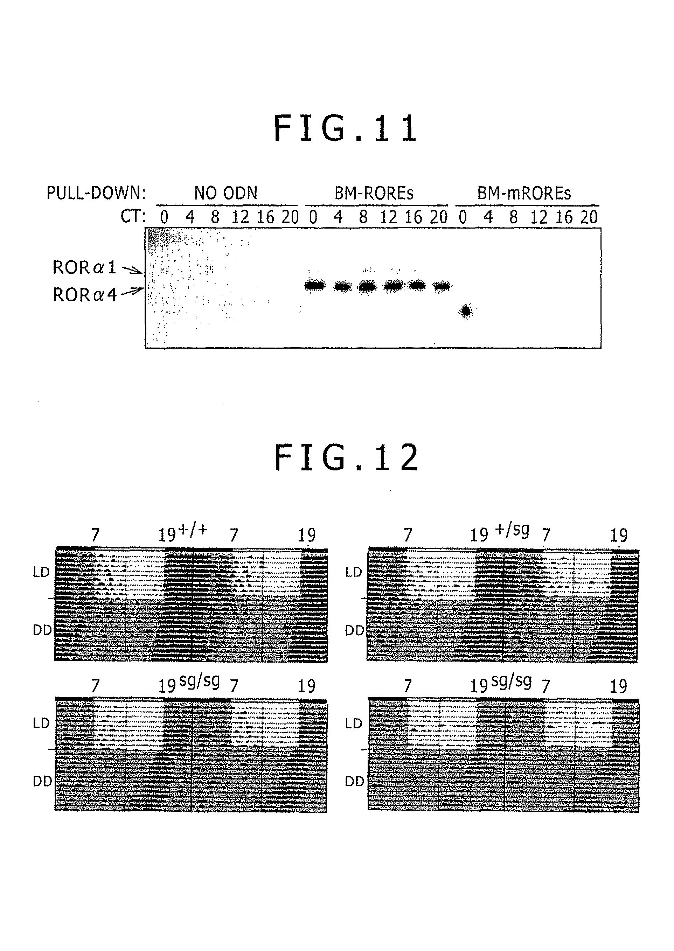

The results are shown in FIG. 11.

In the figure, "no ODN (no oligonucleotide)" indicates the case that no oligonucleotide was bound to biotin (control).

"BM-ROREs" indicates the results of the case that the oligonucleotide with the promoter regions (ROREs) of Bmal1 contained therein was biotinylated.

"BM-mROREs" indicates the results of the case that an oligonucleotide (mutant), which had been prepared by partially modifying the promoter regions (ROREs) of Emal1, was biotinylated. It is to be noted that "m" represents a mutant.

"CT" stands for circadian time. Specifically, it shows the time of the collection of the liver from the mouse, and is indicated in terms of circadian time (time clocked supposing that the starting time of light conditions was 0 hour).

"RORα1" and "RORα4" indicate antibodies employed in the western blotting, and show positions where bands appeared.

As shown in FIG. 11, it was possible to confirm bands corresponding to both "RORα1" and "RORα4", respectively, when the oligonucleotide containing the promoter regions (ROREs) of Bmal1 was employed. In contrast, such bands did not appear when the mutant oligonucleotide was employed.

The above results, therefore, strongly suggest that the binding of RORα to the promoter regions (ROREs) of Bmal1 induces the expression of Bmal1.

### [Example 10]

In Example 10, using RORα-gene knockout mutant mice, an actogram was conducted to investigate possible effects of the deletion of the RORα gene on the circadian rhythms of mice.

"Actogram" means a method for continuously recording active time of a mouse by detecting his or her drinking and eating activities.

FIG. 12 shows actograms when subjected to free running under dark conditions after light conditions and the dark conditions were repeated alternately for 12 hours over 15 days.

In the figure, "+/+", "+/sg" and "sg/sg" indicate a wild-type, a heterogeneous RORα knockout type, and a homogeneous RORα knockout type, respectively.

"LD" indicates the environment that the light conditions (light) and dark conditions (dark) were repeated, while "DD" indicates an environment that the dark conditions (dark) were continued, in other word, were allowed run freely.

As illustrated in FIG. 12, when the rearing environment was changed to 24-hour dark conditions, the starting time of active time was advanced with the mutant mice than with the wild-type mouse. In other words, the circadian cycle became shorter. These results suggest that RORα also affects the circadian rhythms of an individual by stimulating the induction of Bmal1 expression.

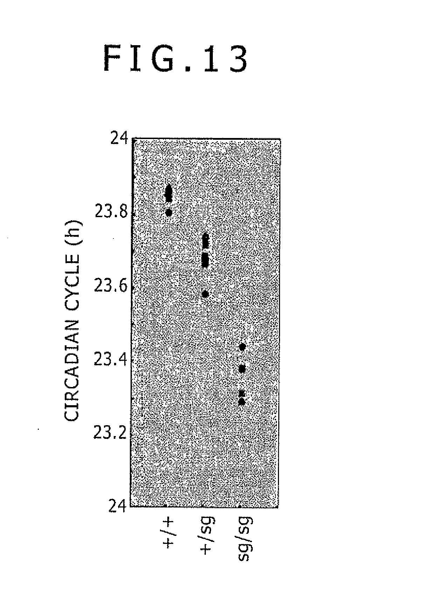

FIG. 13 is a diagram depicting averages of circadian cycles of the mice as acquired after the rearing environment had been changed to 24-hour dark conditions on the basis of the experimental results illustrated in FIG. 12. As shown in FIG. 13, the circadian cycle was about 23.8 hours with the wild-type mouse while the circadian cycle was shortened to about 23.4 hour with the homogeneous RORα-gene knockout mice.

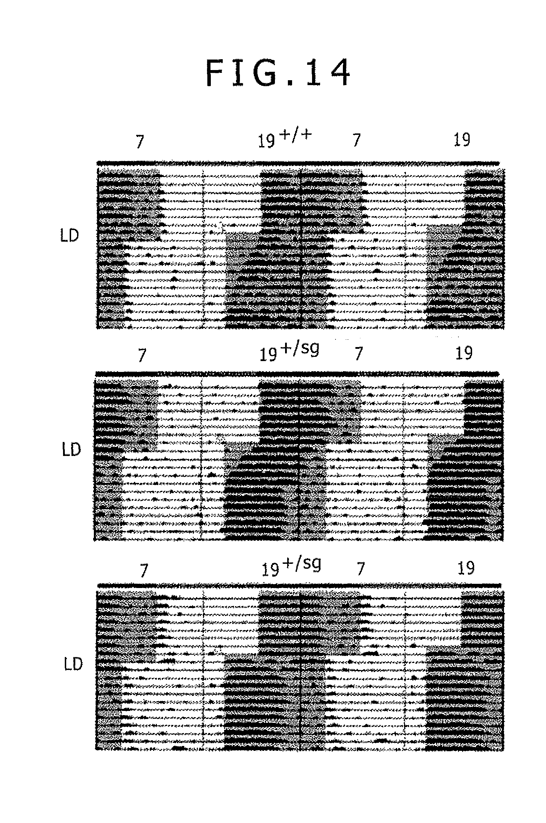

Next, FIG. 14 shows actograms when the starting time of dark conditions was advanced by 4 hours to create a state having a time difference after light conditions and the dark conditions were repeated alternately for 12 hours over 15 days.

The labels in the figure have the same meanings as in FIG. 12.

When the starting time of the dark conditions was advanced by 4 hours, the RORα-gene knockout mice adapted themselves to the new environmental conditions earlier than the wild-type mouse as depicted in FIG. 14.

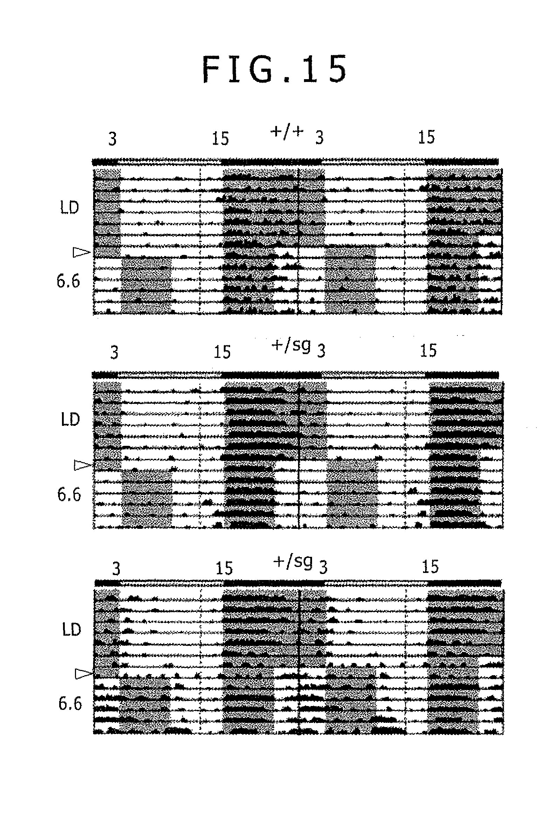

FIG. 15 depicts actograms when the environmental conditions were changed to an environment consisting of light conditions and dark conditions repeated alternately for 6 hours after the light conditions and the dark conditions were repeated alternately for 12 hours over 15 days.

The labels in the figure have the same meanings as in FIG. 12.

As illustrated in FIG. 15, the wild-type mouse retained the circadian rhythms despite the environmental change, while the RORα-gene knockout mice included both of one retained the circadian rhythms and one completely disturbed in the circadian rhythms.

### [Example 11]

In Example 11, an investigation was conducted as to whether or not the induction of Bmal1 expression would be repressed when the expression of RORα is repressed by RNA interference.

An outline of experimental procedure was as follows.
(1) Firstly, a recombinant siRNA expression vector was prepared. From the 849^{th} to 1391^{st} bases in an RORα4-encoding gene sequence, a sequence consisting of 19 bases (SEQ ID NO: 5) was selected. The sequence was then inserted into an siRNA expression vector to prepare the recombinant expression vector.
(2) Similarly to Example 3, etc., the promoter regions of Bmal1 were inserted into the luciferase luminescence vector "pGL3 basic" (product of Promega Corporation) to prepare a recombinant vector.
(3) The recombinant siRNA expression vector and the promoter regions of Bmal1 were next cotransfected into culture cells NIH3T3, and the resulting culture cells were then isolated and subcultured.
(4) A serum shock was then applied to the culture cells to generate a circadian rhythm.
(5) To the resultant culture liquor, luciferin (a substrate for luciferase) was added at 15-minute intervals to measure luciferase activities (luminescence intensities). From circadian variations of the luminescence intensity, a circadian rhythm of Bmal1 transcription was acquired.

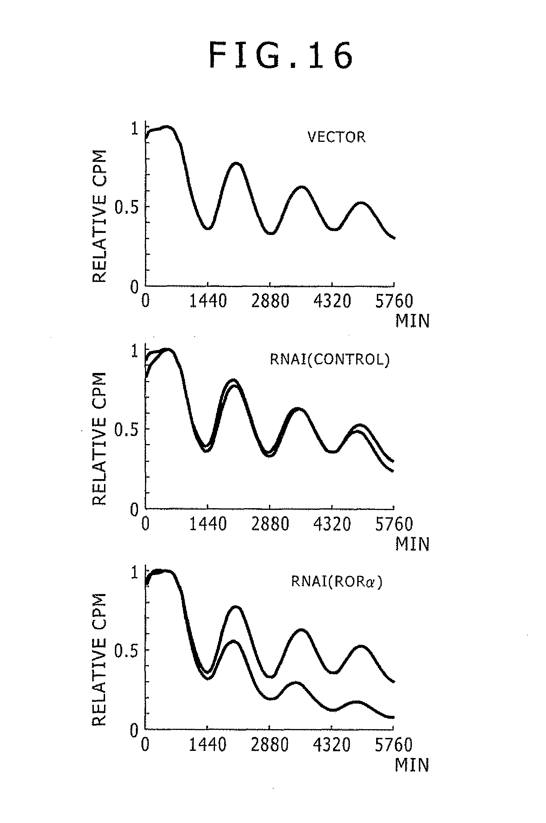

The results are shown in FIG. 16.

In the figure, the abscissa represents the time (unit: min), while the ordinate (relative cpm) represents the luminescence intensity. It is to be noted that luminescence intensities are relative values when the peak of the first luminescence intensity is assumed to be "1".

"vector" indicates the experimental results when the empty vector (pcDNA3) was added in place of the dominant-negative mutant (control).

"RNAi (control)" indicates the use of siRNA irrelevant to the sequence of RORα, and is a control.

"RNAi (RORα)" indicates the use of siRNA having a part of an RORα-encoding base sequence.

As depicted in FIG 16, the amplitude of the induction of Bmal1 expression decreased when siRNA having the part of the RORα-encoding base sequence (in the figure, the lowermost diagram) was used in comparison with the use of the empty vector (the upper curve in the same diagram). The results of this experiment, therefore, indicate that the induction of Bmal1 is also repressed when the expression of ROR is repressed by using RNA interference.

### [Example 12]

In Example 12 and Example 13, investigations were conducted with mouse embryonic fibroblast (MEF) as to whether or not RORα has an effect to stimulate the induction of Bmal1 expression.

In Example 12, circadian variations of the induction of Bmal1 expression were investigated by using an RORα-gene knockout mutant of mouse embryonic fibroblast.

An outline of experimental procedure will hereinafter be described.
(1) In a similar manner as described above, the promoter regions of Bmal1 were firstly inserted into the luciferase luminescence vector "pGL3 basic" (product of Promega Corporation) to prepare a recombinant vector.
(2) The recombinant vector was next transfected into the RORα-gene knockout mutant of mouse embryonic fibroblast, and the resulting culture cells were then isolated and subcultured.
(3) A serum shock was then applied to the culture cells to generate a circadian rhythm.
(4) To the resultant culture liquor, luciferin (a substrate for luciferase) was next added at 15-minute intervals to measure luciferase activities (luminescence intensities). From circadian variations of the luminescence intensity, a circadian rhythm of Bmal1 transcription was acquired.

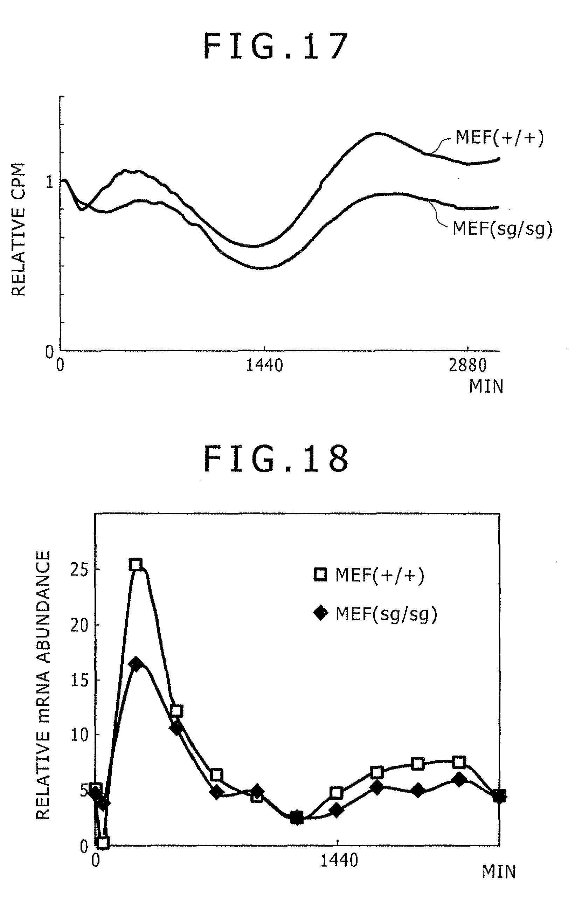

The results are shown in FIG. 17.

In the figure, the abscissa represents the time (unit: min), while the ordinate (relative cpm) represents the luminescence intensity. It is to be noted that luminescence intensities are relative values when the peak of the first luminescence intensity is assumed to be "1".

In the figure, "+/+" and "sg/sg" indicate a wild-type and a heterogeneous RORα knockout type, respectively.

As depicted in FIG. 17, the amplitude of the induction of Bmal1 expression decreased with the RORα-gene knockout mutant of mouse embryonic fibroblast.

### [Example 13]

In Example 13, circadian variations of the expression abundance of Bmal1 in the RORα-gene knockout mutant of mouse embryonic fibroblast were investigated by quantitative realtime RT-PCR.

Subsequent to homogenization of mouse embryonic fibroblast, total RNA was extracted from each organ by using "Promega total SV RNA Isolation Kit" (product of Promega Corporation), and by quantitative realtime RT-PCR, the expression abundance of the Bmal1 gene was measured.

The results are shown in FIG. 18.

In the figure, the abscissa represents the time (unit: min), while the ordinate (relative mRNA abundance) represents the relative expression abundance (expression level). It is to be noted that similarly to Example 1, values of the expression abundance along the ordinate relative values when the expression abundance of the housekeeping gene GAPDH (GlycerAldehyde-3-Phosphate DeHydrogenase) is assumed to be "1".

In the figure, "+/+" and "sg/sg" indicate a wild-type and a heterogeneous RORα knockout type, respectively.

As depicted in FIG. 18, the amplitude of the expression abundance of Bmal1 decreased with the RORα-gene knockout mutant of mouse embryonic fibroblast. It is to be noted that this result was substantially in conformity with the result of FIG. 17.

The results of FIG. 17 and FIG. 18 strongly suggest that even on a cell level, RORα stimulates the induction of Bmal1 expression.

### [Example 14]

In Example 14, an investigation was conducted as to whether or not the induction of Bmal1 expression would also be repressed when the expression of RORα is repressed with an antisense nucleic acid.

An outline of experimental procedure will hereinafter be described.
(1) Firstly, antisense nucleic acid expression vectors were prepared. DNA fragments having the same base sequence as predetermined regions in the RORα-encoding gene were inserted into pcDNA3 to prepare the antisense nucleic acid expression vectors, respectively.
   It is to be noted that as the DNA fragments, three DNA fragments, that is, a fragment having the sequence of from the 65^{th} to 574^{th} bases, a fragment having the sequence of from the 599^{th} to 1122^{nd} bases and a fragment having the sequence of from the 849^{th} to 1391^{st} bases, all in the RORα-encoding gene, were used.
(2) In a similar manner as in Example 3, etc., the promoter regions of Bmal1 were inserted into the luciferase luminescence vector "pGL3 basic" (product of Promega Corporation) to prepare a recombinant vector.
(3) The antisense expression vector and the recombinant vector with the promoter regions of Bmal1 inserted therein were next cotransfected into culture cells NIH3T, and the resulting culture cells were then isolated and subcultured.
(4) A serum shock was then applied to the culture cells to generate a circadian rhythm.
(5) To the resultant culture liquor, luciferin (a substrate for luciferase) was next added at 15-minute intervals to measure luciferase activities (luminescence intensities). From circadian variations of the luminescence intensity, a circadian rhythm of Bmal1 transcription was acquired.

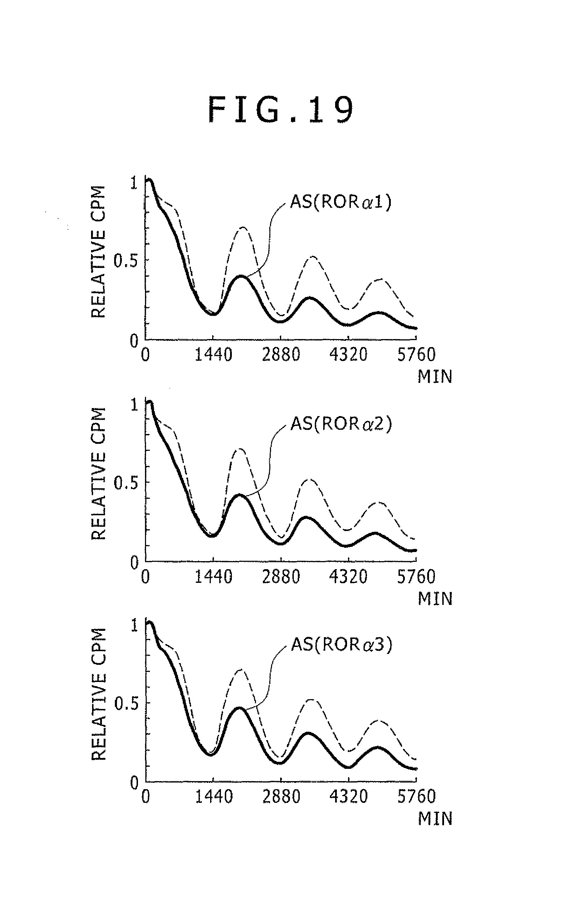

The results are shown in FIG. 19.

In the figure, the abscissa represents the time (unit: min), while the ordinate (relative cpm) represents the luminescence intensity. It is to be noted that luminescence intensities are relative values when the peak of the first luminescence intensity is assumed to be "1".

"AS(RORα1)" indicates that as an antisense nucleic acid, a fragment having a sequence of from the 65^{th} to 574^{th} bases in the RORα-encoding gene was used, "AS(RORα2)" indicates that as an antisense, a fragment having a sequence of from the 599^{th} to 1122^{nd} bases in the RORα-encoding gene was used, and "AS(RORα3)" indicates that as an antisense, a fragment having a sequence of from the 849^{th} to 1391^{st} bases in the RORα-encoding gene was used.

As shown in FIG. 19, the amplitude of the induction of Bmal1 expression was also suppressed by repressing the expression of RORα with any one of the antisense nucleic acids.

### [Example 15]

As mentioned above, it had been ascertained that the expression of RORα is promoted under hypoxia. In Example 15, an investigation was, therefore, conducted as to whether or not the induction of Bmall expression would be promoted under hypoxia.

An outline of experimental procedure will hereinafter be described.
(1) In a similar manner as described above, the promoter regions of Bmal1 were firstly inserted into the luciferase luminescence vector "pGL3 basic" (product of Promega Corporation) to prepare a recombinant vector.
(2) The recombinant vector was next transfected into the RORα-gene knockout mutant of mouse embryonic fibroblast, and the resulting culture cells were then isolated and subcultured.
(3) A serum shock was then applied to the culture cells to generate a circadian rhythm.
(4) After 0.01 mM CoCl₂ was added to the resultant culture liquor, luciferin (a substrate for luciferase) was added at 15-minute intervals to measure luciferase activities (luminescence intensities). From circadian variations of the luminescence intensity, a circadian rhythm of Bmal1 transcription was acquired.

It is to be noted that hypoxia can be created by culturing cells under conditions with added cobalt chloride (CoCl₂).

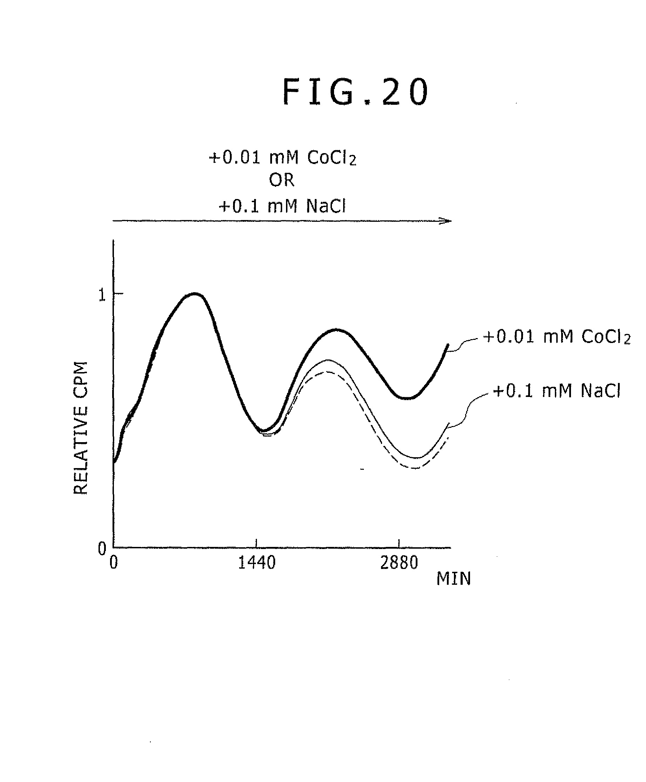

The results are shown in FIG. 20.

In the figure, the abscissa represents the time (unit: min), while the ordinate (relative cpm) represents the luminescence intensity. It is to be noted that luminescence intensities are relative values when the peak of the first luminescence intensity is assumed to be "1".

"+0.01 mM CoCl₂" indicates the addition of 0.01 mM CoCl₂ to the culture liquor, while "+0.01 mM NaCl" indicates the addition of 0.01 mM NaCl (control) in place of 0.01 mM CoCl₂.

The results of FIG. 20 indicate that the induction of Bmal1 expression is also promoted under hypoxia.

### [Industrial Applicability]

The present invention is useful in that it has clarified unelucidated aspects in the control mechanism of circadian rhythms. The present invention is, therefore, applicable as preventives and curatives for diseases affecting the circadian rhythm control mechanism, for example, diseases such as cancer, sleep disorders, jet lag, vigilance disorders, insomnia, vegetative syndromes, depression, and senile dementia. Further, the present invention is also applicable to DNA chips, protein chips, markers for detecting modulations of circadian rhythms, screening methods and screening kits for anticancer agents or time-lag regulating agents, and the like.

### [Sequence Listing]

Other aspects and features of the invention are set out in the following numbered clauses:
1 RORα capable of stimulating an induction of Bmal1 expression.
2 RORα according to clause 1, wherein said expression is promoted under hypoxia.
3 RORα according to clause 1, which acts competitively with REV-ERBα or REV-ERBβ.
4 RORα according to clause 1, which stimulates binding between BMAL1 and CLOCK.
5 RORα according to clause 4, which stimulates an induction of Per gene and/or Cry gene expression.
6 An anticancer agent comprising at least a substance capable of repressing RORα according to clause 1.
7 An anticancer agent according to clause 6, wherein said substance is an anti-RORα antibody.
8 An anticancer agent according to clause 6, wherein said substance is siRNA capable of repressing RORα expression.
9 An anticancer agent according to clause 6, wherein said substance is an antisense nucleic acid.
10 A jet-lag regulating agent comprising at least a substance capable of repressing RORα according to clause 1.
11 A jet-lag regulating agent according to clause 10, wherein said substance is an anti-RORα antibody.
12 A jet-lag regulating agent according to clause 10, wherein said substance is siRNA capable of repressing RORα expression.
13 A jet-lag regulating agent according to clause 10, wherein said substance is an antisense nucleic acid.
14 A DNA chip comprising at least a nucleic acid, which has a base sequence of a gene encoding RORα according to clause 1, or a nucleic acid, which has a part of said base sequence of said gene, immobilized thereon.
15 A protein chip comprising at least an antibody, which specifically binds to RORα according to clause 1, immobilized thereon.
16 Use of RORα according to clause 1 as a marker for detecting a modulation of circadian rhythm.
17 A screening method for an anticancer agent, which comprise searching for a substance capable of repressing RORα according to clause 1.
18 A screening method for a jet-lag regulating agent, which comprise searching for a substance capable of repressing RORα according to clause 1.

## Claims

1. Use of RORα as a marker for detecting a modulation of circadian rhythm.

2. The use according to claim 1 wherein RORα directly activates transcription of Bmal1 through conserved RORα response elements that are required for cell-autonomous transcriptional oscillation of Bmal1 Mrna.

3. The use according to claim 1 or claim 2 wherein an interaction between RORα and RORE1 and/or RORE2 is detected.

4. The use according to claim 1 wherein RORα is essential for the regulation of circadian rhythm of Bmal1 and
1) RORE1 is necessary for the generating of period of circadian rhythm
2) RORE2 is necessary for the regulation of amplitude of circadian rhythm.

5. A screening method for an anticancer agent, which comprises searching for a substance capable of modulating an interaction between RORα and RORE1 and/or RORE2.

6. A screening method for a jet-lag regulating agent, which comprises searching for a substance capable of modulating an interaction between RORα and RORE1 and/or RORE2.
